# EUROPÄISCHE PATENTANMELDUNG

(11) **EP 2 653 531 A1**
(43) Veröffentlichungstag der Anmeldung: **23.10.2013**
(21) Anmeldenummer: 12164553.5
(22) Anmeldetag: 18.04.2012
(51) Int. Cl.: C12M 1/12, C12M 3/06, B01L 3/08

(54) **Kulturanordnung**

(71) Anmelder: Oxyphen AG, 8620 Wetzikon (CH)
(72) Erfinder: Herrmann, Walter, 8865 Bilten (CH); Heusser-Nieweg, 8626 Ottikon (CH)
(74) Vertreter: Clerc, Natalia

(57) **Zusammenfassung**

Eine Kulturanordnung weist ein Kulturgefäss (3) mit einer Aufnahmeöffnung (38) zur Aufnahme eines eine Kulturmembran tragenden Kultureinsatzes (2) auf, wobei der Kultureinsatz (2) in einem Träger (1) gehalten ist. Der Träger (1) weist eine rahmenförmige Struktur zum Tragen des Kultureinsatzes (2) auf. Er ist im Innern des Kulturgefässes (3) angeordnet, wobei sich der Träger (1) von den Seitenwänden des Kulturgefässes (3) und/oder vom Boden (30) des Kulturgefässes erstreckt. Diese Kulturanordnung ermöglicht eine grosse Flexibilität in Form und Grösse der Kulturmembranen. Insbesondere lassen sich grossflächige Kulturmembranen verwenden.

## Beschreibung

Die Erfindung betrifft eine Kulturanordnung für Zell- und Gewebekulturen gemäss Oberbegriff des Patentanspruchs 1, eine Einsatzeinheit zur Verwendung in einer derartigen Kulturanordnung gemäss Oberbegriff des Patentanspruchs 8 und einen Träger zur Verwendung in einer derartigen Kulturanordnung gemäss Oberbegriff des Patentanspruchs 11.

### STAND DER TECHNIK

Derartige Kulturanordnung für Zell- und Gewebekulturen dienen insbesondere zum Kultivieren und Testen von verschiedenen Arten von Zellen, wie zum Beispiel von diversen Epithelzellen und Hautsubstituten.

In den letzten Jahren haben die Aktivitäten im Bereich der Kultivierung von Zellen und Geweben auf Kultureinsätzen mit Polymermembranen stetig zugenommen, zum Beispiel in der Herstellung von Hautsubstituten zum Testen des Effekts von Chemikalien, zum Testen von Kosmetik- oder Hautpflegeprodukten und/oder von pharmazeutischen Produkten auf diversen Zelltypen. Des Weiteren dienen derartige Zellkulturanordnungen zum Kultivieren oder Erzeugen von Hautsubstituten für Hauttransplantationen.

WO 2006/131123 zeigt eine In-Vitro-Kulturanordnung mit einer Kulturschale, mehreren Kultureinsätzen, einem Kultureinsatzträger und einem Deckel. Der Kultureinsatzträger ist auf dem Rand des Gefässes abgestützt und der Deckel stützt sich auf dem Rand des Trägers ab. Der Träger ist im Wesentlichen eine Platte mit Aussparungen für die Kultureinsätze sowie mit Pipettenöffnungen in den Ecken. Nachteilig an dieser Anordnung ist, dass der Träger zwischen der Schale und dem Deckel angeordnet ist und somit die Gesamthöhe der Anordnung vergrössert wird. Ebenfalls lässt sich diese Art von Trägem lediglich bei schalenartigen Kulturgefässen einsetzen.

US 5 795 775 offenbart eine Kulturanordnung mit einem Kulturgefäss, einem Kultureinsatz und einem Deckel. Die Grösse des einsetzbaren Kultureinsatzes ist stark abhängig von der Grösse des Kulturgefässes.

EP 0 590 513 beschreibt eine Kulturanordnung mit einem Kulturgefäss, einem Kultureinsatz und einem Deckel, wobei die Kultureinsätze eine Vielzahl von Membrangeometrien aufweisen. Sie sind beispielsweise kreisförmig, oval, drei-, vier- oder achteckig.

EP 0 606 651 zeigt eine Kulturanordnung mit einem Kulturgefäss, einem Kultureinsatz und einem Deckel, wobei die Kultureinsätze an der Unterseite Füsse oder Abstützelemente aufweisen.

Die bekannten Kulturanordnungen sind im Allgemeinen auf eine einzige Form und/oder Grösse einer Membran ausgerichtet. Wird eine andere Form oder Grösse der Membran verwendet, so muss die gesamte Anordnung inklusive des Kulturgefässes ausgetauscht werden.

### DARSTELLUNG DER ERFINDUNG

Es ist deshalb eine Aufgabe der Erfindung, eine Kulturanordnung zur Verfügung zu stellen, welche bei gleichbleibendem Kulturgefäss eine erhöhte Flexibilität in der Wahl der Kulturmembran ermöglicht.

Diese Aufgabe wird durch eine Kulturanordnung mit den Merkmalen des Patentanspruchs 1, durch eine Einsatzeinheit mit den Merkmalen des Patentanspruchs 7 sowie durch einen Träger mit den Merkmalen des Patentanspruchs 11 gelöst.

Die erfindungsgemässe Kulturanordnung weist ein Kulturgefäss mit einer Aufnahmeöffnung zur Aufnahme eines eine Kulturmembran tragenden Kultureinsatzes auf, wobei der Kultureinsatz in einem Träger gehalten ist. Der Träger weist eine rahmenförmige Struktur zum Tragen des Kultureinsatzes auf und ist im Innern des Kulturgefässes angeordnet. Der Träger erstreckt sich dabei von den Seitenwänden des Kulturgefässes und/oder vom Boden des Kulturgefässes.

Durch die Anordnung des Trägers im Innern des Kulturgefässes, durch seine Halterung an den Seitenwänden bzw. durch seine Auflage auf dem Boden des Kulturgefässträgers sowie durch seine rahmenförmige Struktur ermöglicht er die Halterung von relativ grossen Kulturmembranen. Des Weiteren lässt sich die Formgebung seiner rahmenförmigen Struktur frei wählen, so dass beliebig geformte Kulturmembranen eingesetzt werden können. Dadurch lässt sich derselbe Typus Kulturgefässe mit verschiedenartig geformten Trägem verwenden. Mit Typus sind hier Kulturgefässe derselben Grösse und Form gemeint.
Vorzugsweise ist der Träger beabstandet zum Rand der Aufnahmeöffnung angeordnet; d.h. er liegt tiefer im Innern des Gefässes. Dies hat den Vorteil, dass das Gefäss direkt mit einem Deckel verschliessbar ist, ohne dass der Träger dazwischen liegt. Falls das Gefäss dicht geschlossen werden muss, erhöht dies die Dichtheit und vereinfacht die Erstellung einer dichten Verbindung.

Der Träger kann im Gefäss angeformt und somit einstückig mit diesem ausgebildet sein. In einer bevorzugten Ausführungsform ist der Träger jedoch ein Einsatzelement, welches in das Kulturgefäss einlegbar ist, wobei der Träger vorzugsweise lagefixiert im Kulturgefäss gehalten ist. Die Verwendung eines Einsatzelements erhöht die Flexibilität in der Wahl der Kultureinsätze und Membranen. Ist der Träger lagefixiert angeordnet, so kann er sich beim Transport der Kulturanordnung nicht verschieben und die Membran wird nicht beeinträchtigt.

Der Träger kann sich auf Vorsprüngen der Seitenwände und/oder des Bodens abstützen und/oder klemmend zwischen den Seitenwänden gehalten sein. In einer bevorzugten Ausführungsform ist der Träger jedoch innerhalb des Kulturgefässes auf dessen Boden abstützend angeordnet. Diese Anordnung erleichtert das Einführen des Kultureinsatzes. Des Weiteren optimiert sie die Grösse der frei zur Verfügung stehenden Aufnahmefläche des Trägers. Ist der Träger ein Einsatzelement, so bietet diese Anordnung die einfachste Art und Weise, um den Träger in das Gefäss einzulegen und wieder daraus zu entfernen. Damit der Träger beabstandet vom Boden angeordnet ist und somit die Kulturflüssigkeit frei um die Kulturmembran herum fliessen kann, weist der Träger an seiner rahmenförmigen Struktur vorzugsweise Füsse auf, mit welchen der Träger auf dem Boden abgestützt ist. Alternativ oder zusätzlich weist das Kulturgefäss am Boden und/oder an den Seitenwänden angeformte Vorsprünge auf, auf welchen der Träger abgestützt ist.

Alternativ oder zusätzlich ist der Träger innerhalb des Kulturgefässes durch Klemmmittel und/oder durch seitliche Deformation des Trägers klemmbar gehalten. Die Klemmmittel können beispielsweise Federelemente sein, welche am Gefäss oder am Träger angeformt sind. Vorzugsweise sind sie am Träger angeformt. Die Federelemente können zum Beispiel Blattfedern oder Druckfedern, oder Elemente aus Gummi sein.

Die erfindungsgemässe Einsatzeinheit weist vorzugsweise einen Träger und einen Kultureinsatz auf, wobei der Träger eine rahmenförmige Struktur aufweist, wobei der Kultureinsatz in dieser rahmenförmigen Struktur gehalten ist. Erfindungsgemäss sind an der rahmenförmigen Struktur des Trägers vorstehende Füsse zur Auflage auf einem Boden des Kulturgefässes und/oder seitliche Klemmmittel zur seitlichen Fixierung innerhalb des Kulturgefässes angeordnet.

In einer bevorzugten Ausführungsform sind in der Einsatzeinheit vorstehende Nasen am Kultureinsatz und Nuten zur Aufnahme der Nasen am Träger vorhanden, um den Kultureinsatz lagefixiert im Träger zu halten. Vorzugsweise ist der Kultureinsatz bis auf die Verbindung zwischen Nase und Nut (auch Feder-Nut-Verbindung genannt) und einer allfälligen oberen Auflagefläche vom Träger beabstandet in diesem angeordnet. Dies erleichtert das Einführen und Entfernen des Kultureinsatzes in und aus dem Träger.

Der erfindungsgemässe Träger weist vorzugsweise eine rahmenförmige Struktur auf zur Aufnahme des Kultureinsatzes, wobei an der rahmenförmigen Struktur vorstehende Füsse zur Auflage auf einem Boden des Kulturgefässes und/oder seitliche Klemmmittel zur seitlichen Fixierung innerhalb des Kulturgefässes angeordnet sind.

In einer bevorzugten Ausführungsform sind an der rahmenförmigen Struktur nach aussen vorstehende Abstandhalter zur Abstützung an den Wänden des Kulturgefässes angeordnet. Dies erleichtert das Einführen und Entfernen des Trägers in bzw. aus dem Gefäss. Sind die Abstandhalter federnd ausgebildet oder gemeinsam mit den anschliessenden Seitenwänden des Trägers federn ausgebildet, so verbessern sie die Halterung des Trägers innerhalb des Gefässes.

Vorzugsweise sind auf der Innenseite der rahmenförmigen Struktur Nuten zur Aufnahme des Kultureinsatzes vorhanden, wobei die Abstandhalter an den mit diesen Aufnahmeöffnungen versehenen Stellen der rahmenförmigen Struktur angeordnet sind. Dadurch wird ein gegenseitiges Verklemmen der Kultureinsatzes, des Trägers und des Gefässes verhindert. Der Kultureinsatz lässt sich ohne grössere Kraftaufwendung wieder aus dem Gefäss entfernen. Dies schont die Kulturmembran.

Vorzugsweise ist auf der Aussenseite der rahmenförmigen Struktur mindestens eine Ausnehmung zum manuellen oder maschinellen Ergreifen des Trägers vorhanden ist. Diese Ausnehmung kann in den Abstandhaltern ausgebildet sein, durch entsprechende Formgebung der Seitenwand der Trägers oder durch andere Mittel gebildet sein.

In einer bevorzugten Ausführungsform weist der Träger eine einzige Aufnahmeöffnung zur Aufnahme eines einzigen Kultureinsatzes auf, wobei die einzige Aufnahmeöffnung durch die rahmenförmige Struktur gebildet ist. Dadurch lässt sich eine relativ grosse Kulturmembran verwenden. Dies ist insbesondere bei Hautsubstituten für Hauttransplantationen wünschenswert.

Weitere bevorzugte Ausführungsformen gehen aus den abhängigen Patentansprüchen hervor.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine Explosionszeichnung einer erfindungsgemässen Kulturanordnung in einer perspektivischen Darstellung gemäss einer ersten Ausführungsform;
- Fig. 2: einen vertikalen Schnitt durch die Kulturanordnung gemäss Figur 1 im zusammengebauten Zustand;
- Fig. 3: eine Draufsicht auf einen Träger mit einem eingesetzten Kultureinsatz der Kulturanordnung gemäss Figur 1;
- Fig. 4: einen vertikalen Schnitt entlang der Linie A-A der Figur 3;
- Fig. 5: eine dreidimensionale Darstellung des Kultureinsatzes gemäss Figur 1;
- Fig. 6: eine dreidimensionale Darstellung des Trägers gemäss Figur 1;
- Fig. 7: eine Draufsicht des Träger gemäss Figur 1;
- Fig. 8: einen vertikalen Schnitt durch eine zweite Ausführungsform der erfindungsgemässen Kulturanordnung;
- Fig. 8a: eine vergrösserte Darstellung eines Ausschnitts gemäss Figur 8;
- Fig. 9: einen vertikalen Schnitt durch eine dritte Ausführungsform der erfindungsgemässen Kulturanordnung und
- Fig. 9a: eine vergrösserte Darstellung eines Ausschnitts gemäss Figur 9.

### BESCHREIBUNG BEVORZUGTER AUSFÜHRUNGSFORMEN

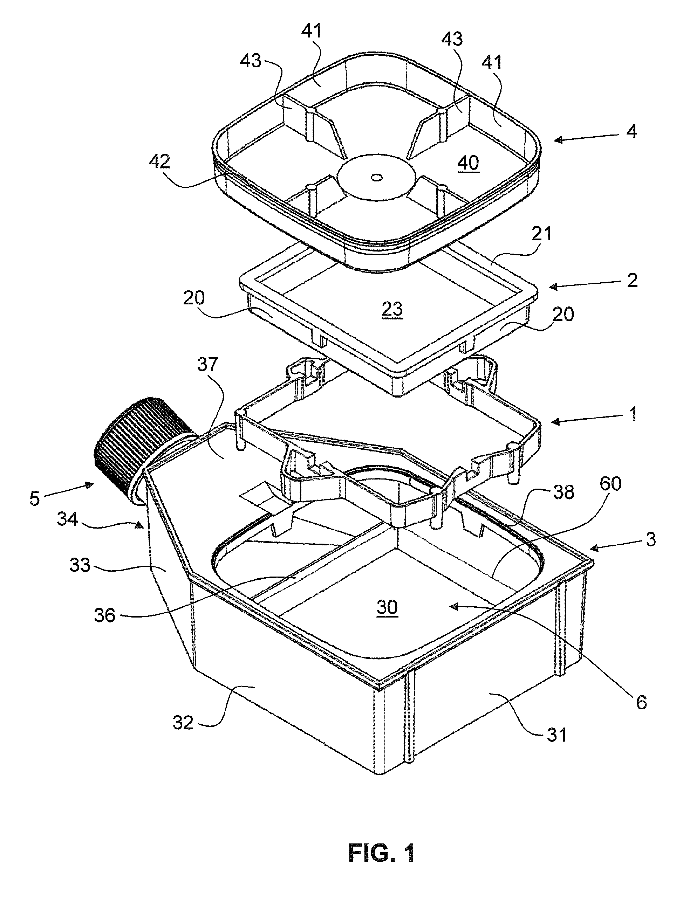

Figur 1 zeigt ein bevorzugtes Beispiel einer erfindungsgemässen Kulturanordnung. Die Kulturanordnung weist ein Zell- oder Gewebe-Kulturgefäss 3, hier in Form einer Kulturflasche 3 auf, in welche eine Flüssigkeit 6 einfüllbar ist. Die Kulturflasche 3 liegt üblicherweise im Gebrauchszustand auf einer Seitenwand auf. Die nachfolgend verwendeten Begriffe wie oben, unten und seitlich beziehen sich auf diesen Gebrauchszustand.

Die Kulturflasche 3 weist einen oberen Deckel 4 und einen seitlichen Verschluss 5 auf. Die Kulturanordnung umfasst ferner einen Träger 1, welcher in die Kulturflasche 3 eingesetzt ist, und einen Kultureinsatz 2, der in diesen Träger 1 eingesetzt ist.

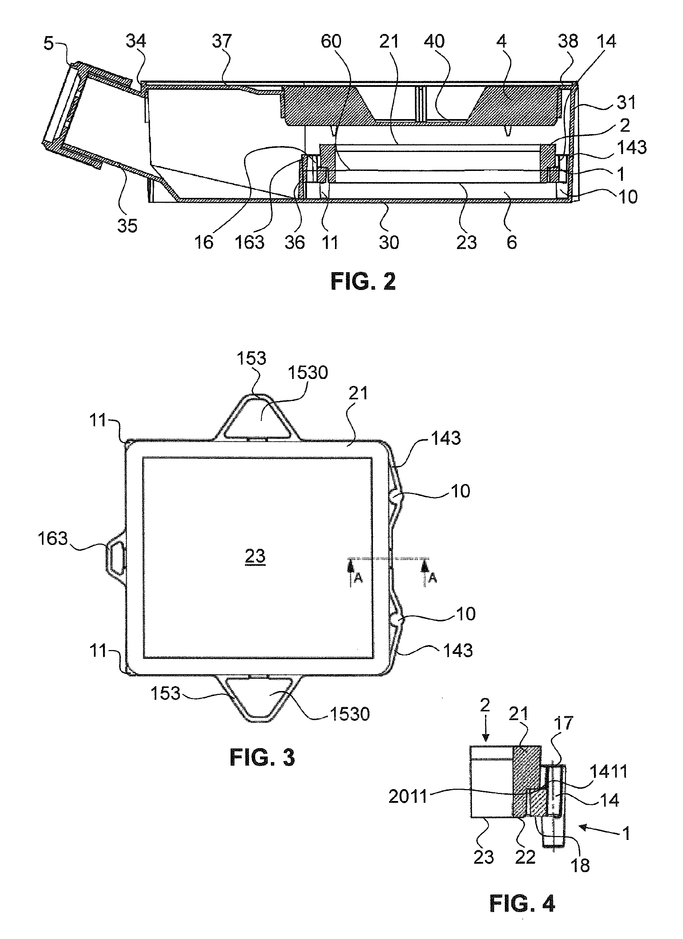

Die Figuren 1 und 2 zeigen eine Flasche 3 wie sie üblicherweise für das Wachstum von Zellen, Geweben oder Mikroorganismen in einer Flüssigkeit 6, wie zum Beispiel einer Nährlösung, verwendet wird. Die Kulturflasche 3 ist so ausgebildet, dass sie einen vollständigen Zugriff auf ihr Inneres erlaubt, ohne dass das Nutzvolumen der Kulturflasche verringert wird.

Die Kulturflasche 3 weist in diesem Beispiel einen grundsätzlich hexagonalen Behälter auf, welcher aus einem geeigneten Material, wie geformtem transparentem Kunststoff, wie zum Beispiel Polyvinylchlorid, vorzugsweise Polystyrol, besteht. Vorzugsweise ist sie einstückig ausgebildet.

Die Kulturflasche 3 beinhaltet einen im Wesentlichen planen Boden 30, welcher von einer Mehrzahl von aufrechtstehenden Wänden 31, 32, 33, 34 umgrenzt ist. Diese Wände beinhalten ein Paar von mehrheitlich parallel zueinander verlaufenden und voneinander beabstandeten länglichen Seitenwänden 32, welche durch eine zu diesen Seitenwänden 32 transversalen, d.h. senkrecht, quer oder schräg zu diesen Seitenwänden 32 angeordneten, Rückwand 31 miteinander verbunden sind. Diese Wände 31, 32 sind senkrecht oder winklig auf dem Boden 30 angeordnet. Der Rückwand 31 liegt eine dazu mehrheitlich parallele Vorderwand 34 gegenüber, welche eine geringere transversale Ausdehnung aufweist als die Rückwand 31. In der Vorderwand 34 ist eine Ausgusstülle vorhanden, welche mit dem Verschluss 5 verschlossen ist.

Zwischen den Seitenwänden 32 und der Vorderwand 34 sind Übergangswände 33 angeordnet, welche auf ihrer einen Seite an eine Seitenwand 32 anschliessen und zusammenlaufen, bis sie auf ihrer anderen Seite an der Vorderwand 34 anschliessen.

Im Innern der Kulturflasche 3 ist eine auf dem Boden 30 stehende, zur Rückwand 31 mehrheitlich parallele, die beiden Seitenwände 32 verbindende aufrechtstehende Wand 36 angeordnet, welche eine geringere Höhe aufweist als die anderen aufrechtstehenden Wände 31, 32, 33, 34. Diese innenliegende Wand 36 ist senkrecht oder winklig auf dem Boden 30 angeordnet. Das Flüssigkeitsniveau 60 wird durch die Höhe dieser innenliegenden Wand 36 begrenzt. Die aufrechtstehenden Wände 31, 32, 33, 34 erstrecken sich aufwärts bis auf eine gemeinsame Höhe und bilden eine zum Boden 30 mehrheitlich parallele Ebene.

Eine mehrheitlich ebene obere Wand 37 ist über den aufrechtstehenden Wänden 31, 32, 33, 34 angeordnet, um die Kulturflasche 3 oben abzuschliessen. Diese obere Wand 37 ist trennbar oder fix auf den oberen Enden der aufrechtstehenden Wänden 31, 32, 33, 34 angeordnet. Die obere Wand 37 weist eine Aufnahmeöffnung 38 auf welche sich annähernd vollständig zwischen den Seitenwänden 32, der Rückwand 31 und den Übergangswänden 33 erstreckt und mit einem Deckel 4 dicht verschliessbar ist. Die Aufnahmeöffnung ist hier im Wesentlichen rechteckförmig oder quadratisch ausgebildet, wobei ihre Ecken vorzugsweise abgerundet sind.

Der Träger 1 ist so ausgebildet, dass er durch diese Aufnahmeöffnung 38 in das Innere der Kulturflasche 3 einführbar ist.

Der Deckel 4 weist einen mehrheitlich ebenen Boden 40 auf, welcher von aufrechtstehenden Wänden 41 umgrenzt wird, welche sich bis auf eine gemeinsame Höhe erstrecken und eine zum Boden 40 parallele, nach oben offene, Umrandung bilden. Diese Umrandung passt dichtend in die Aufnahmeöffnung 38 der Kulturflasche 3. Am oberen Ende der Umrandung des Deckels 4 ist ein Flansch 42 angeformt, der in einen Absatz der oberen Wand 37 passt, und die Kulturflasche 3 dichtend oben abschliesst. Senkrecht auf den Boden des Deckels und jede der aufrechtstehenden Wände sind Rippen 43 angeformt, welche gleichzeitig zur Versteifung und als Halteelemente dienen.

Um einen vorderen Zugang zum Inneren der Kulturflasche zu gewährleisten, ist der Vorderwand 34 eine Ausgusstülle oder Hals 35 integral angeformt. Der Hals 35 ist in diesem Beispiel ein mehrheitlich gleichmässiges zylindrisches Element. Es lässt sich mit dem Verschluss 5, hier ein Drehverschluss, verschliessen.

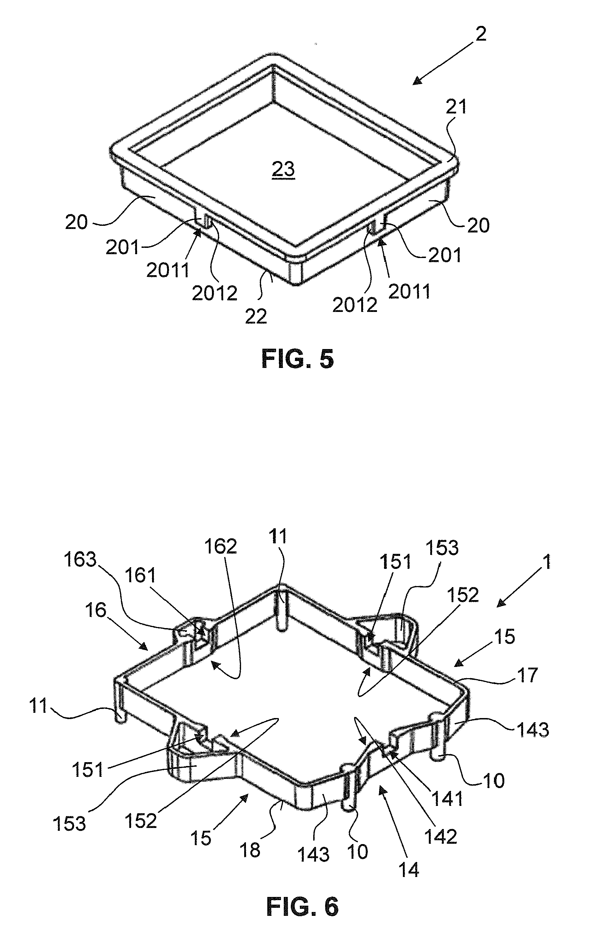

Der Träger 1 weist, wie dies in den Figuren 1 und 6 erkennbar ist, eine grundsätzlich rahmenförmige Struktur auf mit zwei Paaren von sich gegenüberliegenden Wänden 14, 16; 15, 15. Vorzugsweise besteht die Struktur aus einem Kunststoffmaterial, insbesondere aus einem Polymer, wie zum Beispiel Polystyrol, Polypropylen, Polyethylen, ABS, PMMA, Polykarbonat oder anderen geeigneten Materialien. Die Dicke der Wände ist wesentlich geringer als deren Höhen und die Höhen der Wände sind wesentlich geringer als deren Längen.

Die Wände 14, 15, 16 des Trägers 1 begrenzen eine grundsätzlich rechteckige Öffnung an deren oberen und unteren Enden. An einer ersten Trägerwand 14 schliessen sich gegenüberliegende, spiegelbildlich zueinander ausgebildete zweite Trägerwände 15 an. Eine dritte Trägerwand 16 ist der ersten gegenüberliegend angeordnet und schliesst an den beiden zweiten Trägerwänden 15 an. Alle Wände 14, 15, 16 sind vorzugsweise senkrecht oder winklig bezüglich einer horizontalen Ebene angeordnet.

Die erste Trägerwand 14 besteht grundsätzlich aus drei Teilen, wobei die zwei äusseren Teile spiegelbildlich bezüglich des mittleren Teiles zueinander ausgebildet sind. Die beiden äusseren Teile weisen einen ersten Abstandhalter 143 sowie einen innerhalb der ersten Trägerwand 14 angeordneten Fuss 10 auf. Im mittleren Teil der ersten Trägerwand 14 sind eine erste Trägernut 141 sowie eine erste Trägerverdickung 142 ausgebildet. Der mittlere Teil greift in die im Wesentlichen rechteckförmige Öffnung der Trägers 1 ein. Die drei Teile verlaufen vorzugsweise in einem Winkel zueinander.

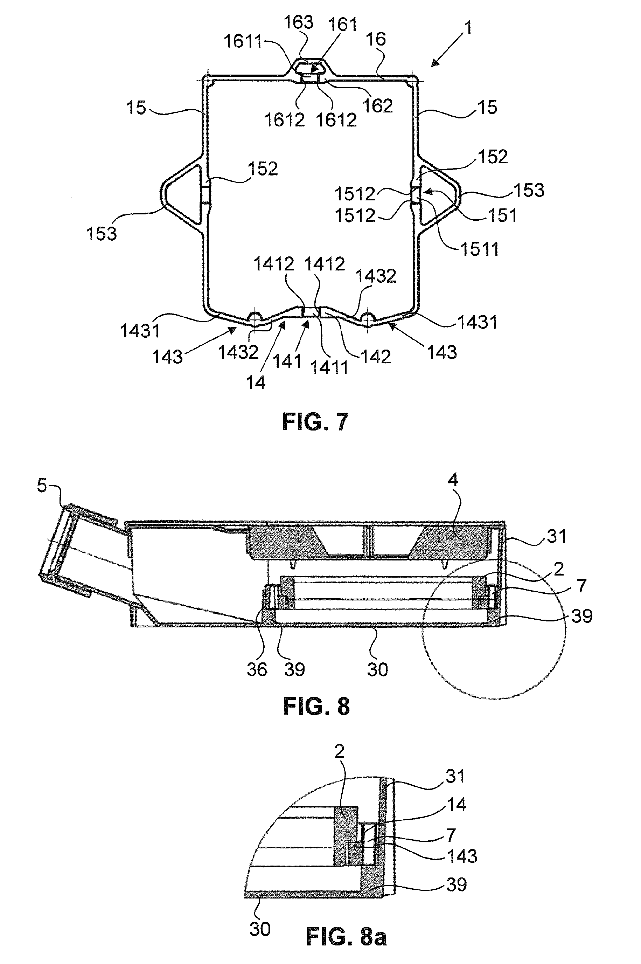

Der erste Abstandhalter 143 ist, wie dies in der Figur 7 erkennbar ist, durch zwei nach aussen gerichtete, aufeinander zulaufende Abschnitte 1431, 1432 des äusseren Trägerabschnittes gebildet. Die innerhalb der ersten Trägerwand 14 angeordneten Füsse 10 sind vorzugsweise in der Schnittlinie der beiden Abschnitte 1431, 1432 angeordnet und bilden die äussersten Elemente der ersten Trägerwand 14. Alternativ sind die Füsse 10 beabstandet zu dieser Schnittlinie und die erste Trägerwand 14 an der Stelle der Schnittlinie bilden die äussersten Begrenzung.

Die zweite und dritte Trägerwand 15, 16 weisen ebenfalls Trägernuten 151, 161 und nach aussen abstehende Abstandhalter 153, 163 auf. Die Trägernut 141, 151, 161 ist jeweils im mittleren Teil der jeweiligen Trägerwand 14, 15, 16 angeordnet und weist eine untere Auflagefläche 1411, 1511, 1611 sowie zwei seitliche Auflageflächen 1412, 1512, 1612 auf. Die Abmessungen zwischen den seitlichen Auflageflächen 1412, 1512, 1612 ist wesentlich geringer als die seitlichen Abmessungen der jeweiligen Trägerwand 14, 15, 16. Die seitlichen Auflageflächen 1412, 1512, 1612 sind senkrecht und parallel zueinander oder vorzugsweise nach unten aufeinander zulaufend ausgebildet.

Die zweiten Abstandhalter 153 der beiden zweiten Trägerwände 15 sind vorzugsweise mittig angeordnet und weisen eine im Wesentlichen trapezförmige Grundform auf, wobei ein langer Schenkel entlang der zweiten Trägerwand 15 verläuft und die Trägernut 151 beinhaltet. Der kurze Schenkel des Trapezes steht frei ab. Das Trapez ist hohl und kann mit einem Werkzeug und/oder mit einem Finger durchsetzt werden. Jede zweite Trägerwand 15 weist vorzugsweise genau einen Abstandhalter 153 auf.

Auch die dritte Trägerwand 16 weist vorzugsweise einen einzigen Abstandhalter 163 auf. Dieser ist vorzugsweise kleiner ausgebildet als die zweiten Abstandhalter 153, weist jedoch vorzugsweise auch eine trapezförmige hohle Grundform auf. Auch hier liegt der lange Schenkel des Trapezes in der dritten Trägerwand 16 und der kurze Schenkel steht frei ab. Im langen Schenkel ist die dritte Trägernute 161 angeordnet. Der dritte Abstandhalter 163 liegt vorzugsweise der Vertiefung der ersten Trägerwand 14 gegenüber und ist mittig in der dritten Trägerwand angeordnet.

Alle Abstandhalter 143, 153, 163 erstrecken sich vorzugsweise über die gesamte Höhe der Rahmenstruktur des Trägers 1.

Die Trägerwände 14, 15, 16 weisen im Bereich der Trägernuten 141, 151, 161 Trägerverdickungen 142, 152, 162 auf. Diese sind nach innen gerichtete Verdickungen der jeweiligen Trägerwand 14, 15, 16 und sie umgeben die jeweilige Trägernut 141, 151, 161. Die seitliche Abmessung der Trägerverdickung 142, 152, 162 ist grösser als die der jeweiligen Trägernut 141, 151, 161. Die Trägerverdickung 142, 152, 162 kompensiert die Schwächung der Trägerwand 14, 15, 16 durch die entsprechende Trägernut 141, 151, 161.

Der Kultureinsatz 2, wie er in den dafür vorgesehenen Träger 1 eingesetzt werden kann, ist in der Figur 5 dargestellt. Der Kultureinsatz 2 hat eine rahmenförmige Struktur, welche zwei Paare von einander gegenüberliegenden Seitenwänden 20 aufweist. Diese Wände 20 sind vorzugsweise senkrecht oder winklig bezüglich einer horizontalen Ebene angeordnet.

Vorzugsweise besteht der Kultureinsatz 2 aus einem Kunststoffmaterial, vorzugsweise aus einem Polymer, wie zum Beispiel Polystyrol, Polypropylen, Polyethylen, ABS, PMMA, Polykarbonat oder anderen geeigneten Materialien. Die Dicke der Seitenwände 20 ist wesentlich geringer als deren Höhen und die Höhen der Seitenwände 20 sind wesentlich geringer als deren Längen.

Eine permeable Kulturmembran 23 ist am unteren Rand 22 des Kultureinsatzes 2 angebracht. Sie besteht aus einem geeigneten Material, zum Beispiel Polyester oder Polykarbonat. Vorzugsweise ist sie mit kapillaren Poren ausgebildet.

Die Seitenwände 20 des Kultureinsatzes 2 begrenzen in diesem Beispiel eine grundsätzlich rechteckige Öffnung an deren oberen und unteren Enden. Die Kulturmembran 23 hat in diesem Beispiel daher auch eine grundsätzlich rechteckige Form.

Ein grundsätzlich vollständig umlaufender oberer Flansch 21 erstreckt sich auswärts vom oberen Ende der Rahmenstruktur des Kultureinsatzes 2. Die Dicke des oberen Flansches 21 ist in etwa gleich wie diejenige der Rahmenstruktur des Kultureinsatzes.

Vom oberen Flansch 21 erstrecken sich Nasen 201 seitlich fluchtend nach unten. Sie sind mit den Seitenwänden 20 einstückig ausgebildet. Die Nasen 201 weisen eine untere Auflagefläche 2011 und seitliche Auflageflächen 2012 auf. Die Abmessung zwischen den seitlichen Auflageflächen 2012 ist wesentlich geringer als die seitlichen Abmessungen des Flansches 21. Die seitlichen Auflageflächen 2012 sind senkrecht und parallel zueinander oder vorzugsweise nach unten aufeinander zulaufend ausgebildet.

Die Form der Nasen 201 des Kultureinsatzes 2 ist vorzugsweise mit der Form der entsprechenden Trägernut 141, 151, 161 abgestimmt.

Die Figur 3 zeigt eine Draufsicht auf den Träger 1 mit dem eingesetzten Kultureinsatz 2. Die seitlichen Abmessungen des Flansches 21 des Kultureinsatzes 2 sind etwas geringer als die entsprechenden Abmessungen des Trägers 1. Der zweite Abstandhalter 153 ist so ausgebildet, dass er eine Ausnehmung 1530 zum manuellen oder maschinellen Ergreifen des Kultureinsatzes 2 aufweist.

Die Figur 4 zeigt einen vertikalen Schnitt entlang der Linie A-A der Figur 3. Der Kultureinsatz 2 steht lediglich durch die Nase 201 des Kultureinsatzes mit dem Träger 1 in Kontakt. Die Positionierung in vertikaler Richtung wird durch die Paarung der unteren Auflagefläche 1411 der Nut 141 mit der unteren Auflagefläche 2011 der Nase 201 realisiert und ist so gestaltet, dass sich ein oberer Trägerrand 17 und der obere Flansch 21 des Kultureinsatzes nicht berühren. Eine Berührung zwischen den Trägerwänden 14, 15, 16 und den entsprechenden Seitenwänden 20 des Kultureinsatzes findet nicht statt.

Analog wird die Positionierung in vertikaler Richtung durch die Paarung der unteren Auflageflächen 1511, 1611 der Nuten 151, 161 mit den unteren Auflageflächen 2011 der Nasen 201 realisiert und eine Berührung zwischen den Trägerwänden 15, 16 mit den entsprechenden Seitenwänden 20 des Kultureinsatzes findet nicht statt. Durch diese Nut-Nasen-Verbindungen 141, 201; 151, 201; 161, 201 wird ein Klemmen beim Einlegen sowie bei der Entnahme des Kultureinsatzes 2 verhindert. Dadurch kann es zu keinen seitlichen Krafteinwirkungen auf die Seitenwänden 20 des Kultureinsatzes kommen. Spannungen in der Kulturmembran 23 des Kultureinsatzes 2 werden vermieden und die Kulturmembran 23 wird nicht beeinträchtigt.

Die Trägerwände 14, 15, 16 sind vorzugsweise so ausgebildet, dass der untere Trägerrand 18 bezüglich dem unteren Rand 22 des Kultureinsatzes 2 im eingebauten Zustand etwas zurücksteht. Alternativ kann der Träger 1 auch so ausgebildet sein, dass der untere Trägerrand 18 mit dem unteren Rand 22 des Kultureinsatzes 2 bündig ausgerichtet ist oder bezüglich diesem etwas vorsteht. Die seitliche Positionierung wird durch die Paarung der seitlichen Auflageflächen 1412, 1512, 1612 der Nuten 141, 151, 161 mit den seitlichen Auflageflächen 2012 der Nasen 201 realisiert.

Wie der Träger 1 mit dem eingesetzten Kultureinsatz 2 gemeinsam in die Kulturflasche 3 mit dem Deckel 4 eingesetzt wird, ist in der Figur 2 dargestellt. Diese zeigt einen vertikalen Schnitt durch die Kulturanordnung gemäss der Figur 1 im zusammengebauten Zustand. Der Träger 1 ist durch Füsse 10, 11 auf dem Boden 30 der Kulturflasche abgestützt, so dass der untere Trägerrand 18 und auch die Membran 23 beabstandet zum Boden 30 angeordnet sind. Eine erste seitliche Positionierung des Trägers 1 wird durch die Paarung der Trägerwände 14, 16 mit den entsprechenden Wänden 31, 36 der Kulturflasche 3 realisiert. Dabei stehen lediglich der erste und der dritte Abstandhalter 143, 163 der entsprechenden ersten Wand 14 und dritten Wand 16 im Kontakt mit den entsprechenden Wänden 31, 36 Flasche3.

Analog und unter Zuhilfenahme von Figur 1 ist es offensichtlich, dass die zweiten Abstandhalter 153 der zweiten Wände 15 des Trägers 1 im Kontakt mit den entsprechenden Seitenwänden 32 der Kulturflasche 3 stehen und die Positionierung in einer, quer zur ersten Positionierung verlaufenden, zweiten Richtung realisieren. Weisen die ersten, zweiten und dritten Abstandhalter 143, 153, 163 des Träger 1 ein Untermass bezüglich der entsprechenden Wänden 31, 32, 36 der Kulturflasche 3 auf, so besteht ein Spiel zwischen der Kulturflasche 3 und dem Träger 1. Weisen die ersten , zweiten und dritten Abstandhalter 143, 153, 163 des Träger 1 ein Übermass bezüglich der entsprechenden Wänden 31, 32, 36 der Kulturflasche 3 auf, so wird der Träger 1 in der Kulturflasche 3 geklemmt.

Der obere Flansch 21 des Kultureinsatzes 2 ist beabstandet zum Rand der oberen Öffnung 38 der Kulturflasche 3 und zum Boden 40 des Deckels 4 angeordnet. Der Träger 1 und der Kultureinsatz 2 sind so aufeinander abgestimmt, dass die Kulturmembran 23 des Kultureinsatzes vollständig von der Flüssigkeit 6 bedeckbar ist.

Die Figur 8 zeigt eine Schnittansicht wie Figur 2, jedoch in einer alternativen Ausführungsform der Kulturflasche 3 und eines Trägers 7. Der Gefässboden 30 der Kulturflasche 3 weist Vorsprünge 39 auf, welche die untere Abstützung des Trägers 7 übernehmen. Der Träger 1 weist keine Füsse 10, 11 auf. Ansonsten weist der Träger 7 vorzugsweise alle in den Figuren 1 bis 7 gezeigten und oben beschriebenen erwähnten Merkmale des Trägers 1 auf. Im speziellen weist der Träger 1 weiterhin die Trägernuten 141, 151, 161, die Trägerverdickungen 142, 152, 162 und die Abstandhalter 143, 153, 163 mit deren Funktionen auf.

Figur 8a zeigt eine Detailansicht der Figur 8, in welcher die Abstützung des Trägers 7 auf den Vorsprüngen 39 vergrössert dargestellt ist.

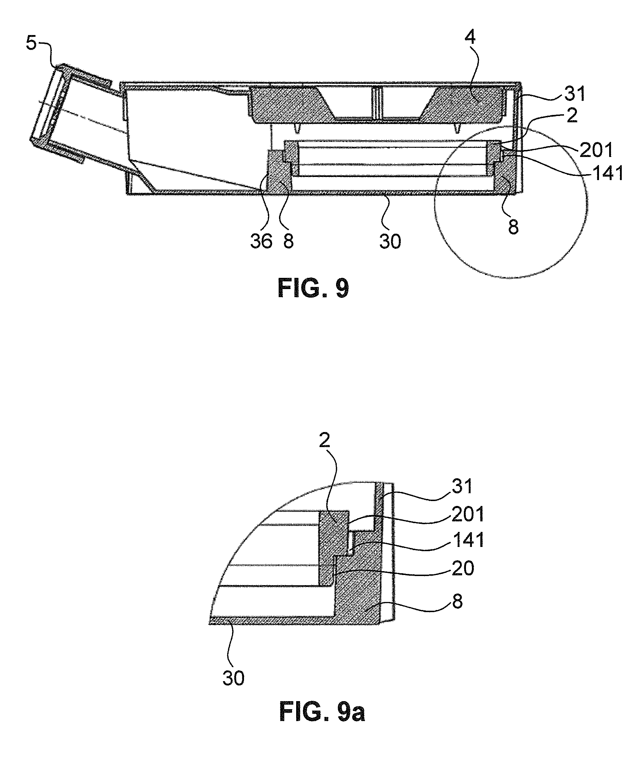

Die Figur 9 zeigt eine Schnittansicht wie Figur 2, jedoch in einer alternativen Ausführungsform der Kulturflasche 3 und eines Trägers 8. Der Träger 8 ist hier einstückig mit dem Gefäss 3 ausgebildet. Er weist keine Füsse 10, 11 zur Abstützung auf. Die Abstandhalter 143, 153, 163 sind ebenfalls in den Gefässwänden 31, 32, 36 integriert. Die Trägerverdickungen 142, 152, 162 zur Verstärkung können grundsätzlich weggelassen werden, können sich jedoch als nützlich erweisen, um die Auflageflächen der Nut 1411, 1412 zu optimieren. Die Nuten 141, 151, 161 des Trägers 8 erstrecken sich nur über einen Teil der Dicke der Träger- bzw. Wand 31, 32, 36. Sie sind so ausgebildet, dass die Nase 201 des Kultureinsatzes 2 mit seitlichem, nach aussen gerichtetem Spiel in die Nuten 141, 151, 161 eingesetzt werden können. Diese sich nicht über die gesamte Dicke der Trägerwand erstreckenden Nuten 141, 151, 161 lassen sich auch in der Ausführung wie sie in den Figuren 1 bis 7 dargestellt ist realisieren.

Figur 9a zeigt eine Detailansicht der Figur 9, in welcher die Abstützung des Kultureinsatzes 2 auf den Träger 8 vergrössert dargestellt ist.

Weitere Ausführungsformen, welche ohne zusätzliche Figuren anschliessend beschrieben werden, sind ebenfalls möglich. So können zum Beispiel Träger mit einer rahmenförmigen Struktur realisiert werden, bei welchen, in der Draufsicht, die Grundform des Rahmens kein Viereck, sondern ein regelmässiges oder unregelmässiges Polygon, mit zum Beispiel, drei, vier, fünf, sechs oder mehr Ecken aufweist. Die Grundform kann auch eine runde, ovale oder kreisartige Form oder eine beliebige Freiform sein. Dasselbe gilt für den Kultureinsatz. Es lassen sich auch Kultureinsätze mit anderen Formen als der Träger aufweist, in diesen Träger anordnen. Die Kulturmembran weist vorzugsweise jeweils die Form des Kultureinsatzes auf. Vorzugsweise sind in allen Ausführungsformen eine einzige Kulturmembran im Kultureinsatz und ein einziger Kultureinsatz im Träger gehalten. Es ist jedoch auch möglich, mehrere Membranen in einen einzigen Einsatz und mehrere Einsätze mit jeweils einer Membran gemeinsam in den Träger anzuordnen.

Der Träger kann auch offen ausgebildet sein und die Anzahl der Seiten des Trägers muss nicht mit der Anzahl der Seiten des Kulturgefässes übereinstimmen.

Auch ist es möglich die beiden Abstützarten des Trägers, Füsse und Bodenerhebungen, zu kombinieren und es sind daher Träger mit ein, zwei, drei oder mehr Füssen möglich.

Die Abstandhalter können ebenfalls weitere Ausführungsformen aufweisen. So können diese offenen Strukturen aufweisen, mehrteilig ausgebildet sein oder mehrmals pro Seite angeformt oder angebracht sein.

Die Anzahl der Nuten pro Seite ist veränderbar und es sind keine, eine, zwei oder mehr Nuten pro Seite möglich.

Alternative Verbindungen zu der in den Figuren 1 bis 9 beschriebenen Nut-Nasen-Verbindung sind ebenfalls möglich. So kann der obere Trägerrand 17 einen teilweise oder vollständig umlaufenden Absatz aufweisen auf und in welchem der Flansch 21 des Kulturträgers angeordnet wird.

Die erfindungsgemässe Kulturanordnung ermöglicht eine grosse Flexibilität in Form und Grösser der Kulturmembranen. Insbesondere lassen sich grossflächige Kulturmembranen verwenden.

### BEZUGSZEICHENLISTE

- 1: Träger
- 10: Erster Fuss
- 11: Zweiter Fuss
- 14: Erste Trägerwand
- 141: Erste Trägernut
- 1411: Untere Auflagefläche
- 1412: Seitliche Auflagefläche
- 142: Erste Trägerverdickung
- 143: Erster Abstandhalter
- 1431: Äusserer Abschnitt
- 1432: Innerer Abschnitt
- 15: Zweite Trägerwand
- 151: Zweite Trägernut
- 1511: Untere Auflagefläche
- 1512: Seitliche Auflagefläche
- 152: Zweite Trägerverdickung
- 153: Zweiter Abstandhalter
- 1530: Ausnehmung
- 16: Dritte Trägerwand
- 161: Dritte Trägernut
- 1611: Untere Auflagefläche
- 1612: Seitliche Auflagefläche
- 162: Dritte Trägerverdickung
- 163: Dritter Abstandhalter
- 17: Oberer Trägerrand
- 18: Unterer Trägerrand

- 2: Kultureinsatz
- 20: Seitenwand
- 21: Oberer Flansch
- 201: Nase

- 2011: Untere Auflagefläche
- 2012: Seitliche Auflagefläche
- 22: Unterer Rand
- 23: Kulturmembran

- 3: Kulturflasche
- 30: Boden
- 31: Rückwand
- 32: Seitenwand
- 33: Übergangswand
- 34: Vorderwand
- 35: Hals
- 36: Innenliegende Wand
- 37: Obere Wand
- 38: Aufnahmeöffnung
- 39: Vorsprung

- 4: Deckel
- 40: Boden
- 41: Wand
- 42: Flansch
- 43: Rippe

- 5: Verschluss
- 6: Flüssigkeit
- 60: Flüssigkeitsniveau

- 7: Alternativer Träger

- 8: Alternativer Träger

## Patentansprüche

1. Kulturanordnung, welche ein Kulturgefäss (3) mit einer Aufnahmeöffnung (38) zur Aufnahme eines eine Kulturmembran tragenden Kultureinsatzes (2) aufweist, wobei der Kultureinsatz (2) in einem Träger (1) gehalten ist, **dadurch gekennzeichnet, dass** der Träger (1) eine rahmenförmige Struktur zum Tragen des Kultureinsatzes (2) aufweist und im Innern des Kulturgefässes (3) angeordnet ist, wobei sich der Träger (1) von den Seitenwänden des Kulturgefässes (3) und/oder vom Boden (30) des Kulturgefässes erstreckt.

2. Kulturanordnung gemäss Anspruch 1, wobei der Träger (1) beabstandet zum Rand der Aufnahmeöffnung (38) angeordnet ist.

3. Kulturanordnung gemäss einem der Ansprüche 1 oder 2, wobei der Träger (1) ein Einsatzelement ist, welches in das Kulturgefäss (3) einlegbar ist, wobei der Träger (1) vorzugsweise lagefixiert im Kulturgefäss (3) gehalten ist.

4. Kulturanordnung gemäss einem der Ansprüche 1 bis 3, wobei der Träger (1) innerhalb des Kulturgefässes (3) auf dessen Boden (30) abstützend angeordnet ist.

5. Kulturanordnung gemäss einem der Ansprüche 1 bis 4, wobei an der rahmenförmigen Struktur Füsse (10, 11) angeordnet sind, wobei der Träger mit diesen Füssen (10, 11) auf dem Boden (30) abgestützt ist und die rahmenförmige Struktur beabstandet zum Boden (30) angeordnet ist.

6. Kulturanordnung gemäss einem der Ansprüche 1 bis 5, wobei das Kulturgefäss (3) am Boden (30) angeformte Vorsprünge (39) aufweist, auf welchen der Träger (1) abgestützt ist.

7. Kulturanordnung gemäss einem der Ansprüche 1 bis 6, wobei der Träger (1) innerhalb des Kulturgefässes (3) durch Klemmmittel und/oder durch seitliche Deformation des Trägers klemmbar gehalten ist.

8. Einsatzeinheit mit einem Träger (1) und einem Kultureinsatz (2) zur Verwendung in einer Kulturanordnung gemäss einem der Ansprüche 1 bis 7, wobei die Kulturanordnung ein Kulturgefäss (3) umfasst, **dadurch gekennzeichnet, dass** der Träger (1) eine rahmenförmige Struktur aufweist, dass der Kultureinsatz (2) in dieser rahmenförmigen Struktur gehalten ist und dass an der rahmenförmigen Struktur vorstehende Füsse (10, 11) zur Auflage auf einem Boden (30) des Kulturgefässes (3) und/oder seitliche Klemmmittel zur seitlichen Fixierung innerhalb des Kulturgefässes (3) angeordnet sind.

9. Einsatzeinheit gemäss Anspruch 8, wobei vorstehende Nasen (201) und Nuten (141, 151, 161) zur Aufnahme der Nasen vorhanden sind, um den Kultureinsatz (2) lagefixiert im Träger (1) zu halten.

10. Einsatzeinheit gemäss Anspruch 9, wobei der Kultureinsatz (2) bis auf die Verbindung zwischen Nasen und Nuten und einer allfälligen oberen Auflagefläche (17) vom Träger beabstandet in diesem angeordnet ist.

11. Träger zur Verwendung in einer Kulturanordnung gemäss einem der Ansprüche 1 bis 7, wobei die Kulturanordnung ein Kulturgefäss (3) und einen Kultureinsatz (2) umfasst, **dadurch gekennzeichnet, dass** der Träger (1) eine rahmenförmige Struktur aufweist zur Aufnahme des Kultureinsatzes (2) und dass an der rahmenförmigen Struktur vorstehende Füsse (10, 11) zur Auflage auf einem Boden (30) des Kulturgefässes (3) und/oder seitliche Klemmmittel zur seitlichen Fixierung innerhalb des Kulturgefässes (3) angeordnet sind.

12. Träger nach Anspruch 11, wobei an der rahmenförmigen Struktur nach aussen vorstehende Abstandhalter (143, 153, 163) zur Abstützung an den Wänden des Kulturgefässes (2) angeordnet sind.

13. Träger nach Anspruch 12, wobei auf der Innenseite der rahmenförmigen Struktur Nuten (141, 151, 161) zur Aufnahme des Kultureinsatzes (2) vorhanden sind und wobei die Abstandhalter (143, 153, 163) an den mit diesen Aufnahmeöffnungen versehenen Stellen der rahmenförmigen Struktur angeordnet sind.

14. Träger nach einem der Ansprüche 11 bis 13, wobei auf der Aussenseite der rahmenförmigen Struktur mindestens eine Ausnehmung (1530) zum manuellen oder maschinellen Ergreifen des Trägers (1) vorhanden ist.

15. Träger nach einem der Ansprüche 11 bis 14, wobei er eine einzige Aufnahmeöffnung zur Aufnahme eines einzigen Kultureinsatzes aufweist, wobei die einzige Aufnahmeöffnung durch die rahmenförmige Struktur gebildet ist.
